# EUROPEAN PATENT APPLICATION

(11) **EP 1 431 396 A1**
(43) Date of publication of application: **23.06.2004**
(21) Application number: 03028086.1
(22) Date of filing: 09.12.2003
(51) Int. Cl.: C12Q 1/52, C12Q 1/54, G01N 33/52, C12N 9/10

(54) **Method for measuring glucosamine-6-phosphate**

(30) Priority: 19.12.2002 US 434615 P
(71) Applicant: F. HOFFMANN-LA ROCHE AG, 4070 Basel (CH)
(72) Inventor: Burghardt, Charles, Mahwah New Jersey 07430 (US); Kochan, Jarema Peter, Verona New Jersey 07044 (US)

(57) **Abstract**

The present invention pertains to methods for measuring the activity of glutamine:fructose 6-phosphate amidotransferase which comprise the steps of (a) forming a mixture of glutamine:fructose 6-phosphate amidotransferase, fructose 6-phosphate, and glutamine; (b) incubating the mixture under physiological conditions to allow the formation of glucosamine 6-phosphate; (c) acetylating the glucosamine 6-phosphate to form N-acetylglucosamine 6-phosphate; (d) reacting the N-acetylglucosamine 6-phosphate with Ehrlich's reagent; and (e) measuring the amount of N-acetylglucosamine 6-phosphate by determining the optical density at 500-610nm of the mixture and comparing the amount of N-acetylglucosamine 6-phosphate with control samples. The present invention also pertains to methods for measuring the inhibitory activity of a test compound on glutamine:fructose 6-phosphate amidotransferase, which comprise the steps of (a) forming a mixture of a test compound, glutamine:fructose 6-phosphate amidotransferase, fructose 6-phosphate, and glutamine;
(b) incubating the mixture under physiological conditions to allow the test compound to inhibit glutamine:fructose 6-phosphate amidotransferase from forming glucosamine 6-phosphate; (c) acetylating the glucosamine 6-phosphate to form N-acetylglucosamine 6-phosphate; (d) reacting the N-acetylglucosamine 6-phosphate with Ehrlich's reagent; and
(e) measuring the amount of N-acetylglucosamine 6-phosphate by determining the optical density at 500-610nm of the mixture and comparing the amount of N-acetylglucosamine 6-phosphate with control samples.

SEQUENCE LISTING

<110>  F. Hoffmann-La Roche

<120>  Methods for measuring activity of Glutamine:Fructose 6-
phosphate Amidotransferase

<130>  21447

<160>  2

<170>  PatentIn version 3.2

<210>  1

<211>  681

<212>  PRT

<213>  Homo sapiens

<220>

<221>  GFAT-alpha

<222>  (1)..(681)

<223>  GenBank accession No. M90516

<400>  1

Met Cys Gly Ile Phe Ala Tyr Leu Asn Tyr His Val Pro Arg Thr Arg

1               5                   10                  15

Arg Glu Ile Leu Glu Thr Leu Ile Lys Gly Leu Gln Arg Leu Glu Tyr

            20                  25                  30

Arg Gly Tyr Asp Ser Ala Gly Val Gly Phe Asp Gly Gly Asn Asp Lys

        35                  40                  45

Asp Trp Glu Ala Asn Ala Cys Lys Thr Gln Leu Ile Lys Lys Lys Gly

    50                  55                  60

Lys Val Lys Ala Leu Asp Glu Glu Val His Lys Gln Gln Asp Met Asp

65                  70                  75                  80

Leu Asp Ile Glu Phe Asp Val His Leu Gly Ile Ala His Thr Arg Trp

                85                  90                  95

Ala Thr His Gly Glu Pro Ser Pro Val Asn Ser His Pro Gln Arg Ser

            100                 105                 110

Asp Lys Asn Asn Glu Phe Ile Val Ile His Asn Gly Ile Ile Thr Asn

        115                 120                 125

Tyr Lys Asp Leu Lys Lys Phe Leu Glu Ser Lys Gly Tyr Asp Phe Glu

    130                 135                 140

Ser Glu Thr Asp Thr Glu Thr Ile Ala Lys Leu Val Lys Tyr Met Tyr

145                 150                 155                 160

Asp Asn Arg Glu Ser Gln Asp Thr Ser Phe Thr Thr Leu Val Glu Arg

                165                 170                 175

Val Ile Gln Gln Leu Glu Gly Ala Phe Ala Leu Val Phe Lys Ser Val

            180                 185                 190

His Phe Pro Gly Gln Ala Val Gly Thr Arg Arg Gly Ser Pro Leu Leu

        195                 200                 205

Ile Gly Val Arg Ser Glu His Lys Leu Ser Thr Asp His Ile Pro Ile

    210                 215                 220

Leu Tyr Arg Thr Gly Lys Asp Lys Lys Gly Ser Cys Asn Leu Ser Arg

225                 230                 235                 240

Val Asp Ser Thr Thr Cys Leu Phe Pro Val Glu Glu Lys Ala Val Glu

                245                 250                 255

Tyr Tyr Phe Ala Ser Asp Ala Ser Ala Val Ile Glu His Thr Asn Arg

            260                 265                 270

Val Ile Phe Leu Glu Asp Asp Asp Val Ala Ala Val Val Asp Gly Arg

        275                 280                 285

Leu Ser Ile His Arg Ile Lys Arg Thr Ala Gly Asp His Pro Gly Arg

    290                 295                 300

Ala Val Gln Thr Leu Gln Met Glu Leu Gln Gln Ile Met Lys Gly Asn

305                 310                 315                 320

Phe Ser Ser Phe Met Gln Lys Glu Ile Phe Glu Gln Pro Glu Ser Val

                325                 330                 335

Val Asn Thr Met Arg Gly Arg Val Asn Phe Asp Asp Tyr Thr Val Asn

            340                 345                 350

Leu Gly Gly Leu Lys Asp His Ile Lys Glu Ile Gln Arg Cys Arg Arg

        355                 360                 365

Leu Ile Leu Ile Ala Cys Gly Thr Ser Tyr His Ala Gly Val Ala Thr

    370                 375                 380

Arg Gln Val Leu Glu Glu Leu Thr Glu Leu Pro Val Met Val Glu Leu

385                 390                 395                 400

Ala Ser Asp Phe Leu Asp Arg Asn Thr Pro Val Phe Arg Asp Asp Val

                405                 410                 415

Cys Phe Phe Leu Ser Gln Ser Gly Glu Thr Ala Asp Thr Leu Met Gly

            420                 425                 430

Leu Arg Tyr Cys Lys Glu Arg Gly Ala Leu Thr Val Gly Ile Thr Asn

        435                 440                 445

Thr Val Gly Ser Ser Ile Ser Arg Glu Thr Asp Cys Gly Val His Ile

    450                 455                 460

Asn Ala Gly Pro Glu Ile Gly Val Ala Ser Thr Lys Ala Tyr Thr Ser

465                 470                 475                 480

Gln Phe Val Ser Leu Val Met Phe Ala Leu Met Met Cys Asp Asp Arg

                485                 490                 495

Ile Ser Met Gln Glu Arg Arg Lys Glu Ile Met Leu Gly Leu Lys Arg

            500                 505                 510

Leu Pro Asp Leu Ile Lys Glu Val Leu Ser Met Asp Asp Glu Ile Gln

        515                 520                 525

Lys Leu Ala Thr Glu Leu Tyr His Gln Lys Ser Val Leu Ile Met Gly

    530                 535                 540

Arg Gly Tyr His Tyr Ala Thr Cys Leu Glu Gly Ala Leu Lys Ile Lys

545                 550                 555                 560

Glu Ile Thr Tyr Met His Ser Glu Gly Ile Leu Ala Gly Glu Leu Lys

                565                 570                 575

His Gly Pro Leu Ala Leu Val Asp Lys Leu Met Pro Val Ile Met Ile

            580                 585                 590

Ile Met Arg Asp His Thr Tyr Ala Lys Cys Gln Asn Ala Leu Gln Gln

        595                 600                 605

Val Val Ala Arg Gln Gly Arg Pro Val Val Ile Cys Asp Lys Glu Asp

    610                 615                 620

Thr Glu Thr Ile Lys Asn Thr Lys Arg Thr Ile Lys Val Pro His Ser

625                 630                 635                 640

Val Asp Cys Leu Gln Gly Ile Leu Ser Val Ile Pro Leu Gln Leu Leu

                645                 650                 655

Ala Phe His Leu Ala Val Leu Arg Gly Tyr Asp Val Asp Phe Pro Arg

            660                 665                 670

Asn Leu Ala Lys Ser Val Thr Val Glu

        675                 680

<210> 2

<211> 682

<212> PRT

<213> Homo sapiens

<220>

<221> GFAT-beta

<222> (1)..(682)

<223> AB016789

<400> 2

Met Cys Gly Ile Phe Ala Tyr Met Asn Tyr Arg Val Pro Arg Thr Arg

1               5                   10                  15

Lys Glu Ile Phe Glu Thr Leu Ile Lys Gly Leu Gln Arg Leu Glu Tyr

            20                  25                  30

Arg Gly Tyr Asp Ser Ala Gly Val Ala Ile Asp Gly Asn Asn His Glu

        35                  40                  45

Val Lys Glu Arg His Ile Gln Leu Val Lys Lys Arg Gly Lys Val Lys

    50                  55                  60

Ala Leu Asp Glu Glu Leu Tyr Lys Gln Asp Ser Met Asp Leu Lys Val

65                  70                  75                  80

Glu Phe Glu Thr His Phe Gly Ile Ala His Thr Arg Trp Ala Thr His

                85                  90                  95

Gly Val Pro Ser Ala Val Asn Ser His Pro Gln Arg Ser Asp Lys Gly

            100                 105                 110

Asn Glu Phe Val Val Ile His Asn Gly Ile Ile Thr Asn Tyr Lys Asp

        115                 120                 125

Leu Arg Lys Phe Leu Glu Ser Lys Gly Tyr Glu Phe Glu Ser Glu Thr

    130                 135                 140

Asp Thr Glu Thr Ile Ala Lys Leu Ile Lys Tyr Val Phe Asp Asn Arg

145                 150                 155                 160

Glu Thr Glu Asp Ile Thr Phe Ser Thr Leu Val Glu Arg Val Ile Gln

                165                 170                 175

Gln Leu Glu Gly Ala Phe Ala Leu Val Phe Lys Ser Val His Tyr Pro

            180                 185                 190

Gly Glu Ala Val Ala Thr Arg Arg Gly Ser Pro Leu Leu Ile Gly Val

        195                 200                 205

Arg Ser Lys Tyr Lys Leu Ser Thr Glu Gln Ile Pro Ile Leu Tyr Arg

    210                 215                 220

Thr Cys Thr Leu Glu Asn Val Lys Asn Ile Cys Lys Thr Arg Met Lys

225                 230                 235                 240

Arg Leu Asp Ser Ser Ala Cys Leu His Ala Val Gly Asp Lys Ala Val

                245                 250                 255

Glu Phe Phe Phe Ala Ser Asp Ala Ser Ala Ile Ile Glu His Thr Asn

            260                 265                 270

Arg Val Ile Phe Leu Glu Asp Asp Asp Ile Ala Ala Val Ala Asp Gly

        275                 280                 285

Lys Leu Ser Ile His Arg Val Lys Arg Ser Ala Ser Asp Asp Pro Ser

    290                 295                 300

Arg Ala Ile Gln Thr Leu Gln Met Glu Leu Gln Gln Ile Met Lys Gly

305                 310                 315                 320

Asn Phe Ser Ala Phe Met Gln Lys Glu Ile Phe Glu Gln Pro Glu Ser
                325                 330                 335
Val Phe Asn Thr Met Arg Gly Arg Val Asn Phe Glu Thr Asn Thr Val
            340                 345                 350
Leu Leu Gly Gly Leu Lys Asp His Leu Lys Glu Ile Arg Arg Cys Arg
        355                 360                 365
Arg Leu Ile Val Ile Gly Cys Gly Thr Ser Tyr His Ala Ala Val Ala
    370                 375                 380
Thr Arg Gln Val Leu Glu Glu Leu Thr Glu Leu Pro Val Met Val Glu
385                 390                 395                 400
Leu Ala Ser Asp Phe Leu Asp Arg Asn Thr Pro Val Phe Arg Asp Asp
                405                 410                 415
Val Cys Phe Phe Ile Ser Gln Ser Gly Glu Thr Ala Asp Thr Leu Leu
            420                 425                 430
Ala Leu Arg Tyr Cys Lys Asp Arg Gly Ala Leu Thr Val Gly Val Thr
        435                 440                 445
Asn Thr Val Gly Ser Ser Ile Ser Arg Glu Thr Asp Cys Gly Val His

    450                 455                 460

Ile Asn Ala Gly Pro Glu Ile Gly Val Ala Ser Thr Lys Ala Tyr Thr

465                 470                 475                 480

Ser Gln Phe Ile Ser Leu Val Met Phe Gly Leu Met Met Ser Glu Asp

                485                 490                 495

Arg Ile Ser Leu Gln Asn Arg Arg Gln Glu Ile Ile Arg Gly Leu Arg

            500                 505                 510

Ser Leu Pro Glu Leu Ile Lys Glu Val Leu Ser Leu Glu Glu Lys Ile

        515                 520                 525

His Asp Leu Ala Leu Glu Leu Tyr Thr Gln Arg Ser Leu Leu Val Met

    530                 535                 540

Gly Arg Gly Tyr Asn Tyr Ala Thr Cys Leu Glu Gly Ala Leu Lys Ile

545                 550                 555                 560

Lys Glu Ile Thr Tyr Met His Ser Glu Gly Ile Leu Ala Gly Glu Leu

                565                 570                 575

Lys His Gly Pro Leu Ala Leu Ile Asp Lys Gln Met Pro Val Ile Met

            580                 585                 590

Val Ile Met Lys Asp Pro Cys Phe Ala Lys Cys Gln Asn Ala Leu Gln
        595                 600                 605
Gln Val Thr Ala Arg Gln Gly Arg Pro Ile Ile Leu Cys Ser Lys Asp
    610                 615                 620
Asp Thr Glu Ser Ser Lys Phe Ala Tyr Lys Thr Ile Glu Leu Pro His
625                 630                 635                 640
Thr Val Asp Cys Leu Gln Gly Ile Leu Ser Val Ile Pro Leu Gln Leu
                645                 650                 655
Leu Ser Phe His Leu Ala Val Leu Arg Gly Tyr Asp Val Asp Phe Pro
            660                 665                 670
Arg Asn Leu Ala Lys Ser Val Thr Val Glu
        675                 680

## Description

The present invention pertains to methods for measuring the activity of glutamine:fructose 6-phosphate amidotransferase and to methods for measuring the inhibitory activity of a test compound on glutamine:fructose 6-phosphate amidotransferase. The present invention also pertains to methods for measuring the formation of uridine-diphosphate-N-acetylglucosamine in extracts from human and animal tissue or cells in tissue cultures, to methods for measuring the inhibitory activity of a test compound on uridine-diphosphate-N-acetylglucosamine formation in cells, and to methods for measuring the inhibitory activity of a test compound on uridine-diphosphate-N-acetylglucosamine formation in a human or an animal.

Methods for analyzing glucosamine 6-phosphate and uridine-diphosphate-N-acetylglucosamine (UDP-N-acetylglucosamine, UDP-GlcNAc) are known. Glucosamine 6-phosphate is the first product of the enzyme glutamine:fructose 6-phosphate amidotransferase (GFAT), which is the rate-limiting enzyme in the hexosamine biosynthetic pathway. UDP-N-acetylglucosamine is the final product in the hexosamine biosynthetic pathway, which is then used, in the N-linked and O-linked glycosylation of proteins. In addition, it is acted upon by O-N-acetylglucosamine transferase to transfer a single N-acetylglucosamine residue onto serine or threonine amino acids. These pathways are important in the regulation of protein glycosylation and have been associated with insulin sensitivity and glucose regulation. The measurement of glucosamine 6-phosphate has been labor intensive using either of two methods for detection: 1) extraction and purification of the sample followed by HPLC/MS analysis (K.A. Robinson, M.L. Weinstein, G.E. Lindenmayer, and M.G. Buse (1995) Diabetes 44 1438-1446); or 2) a spectrophotometric method that requires either boiling the samples in water, or extracting the samples with perchloric acid followed by neutralization, and centrifugation steps. These methods are not amenable to high throughput assays.

This method of glucosamine 6-phosphate determination is used to identify GFAT inhibitors as well as their subsequent optimization. The measurement of UDP-GlcNAc is used to determine the effect of GFAT inhibitors *in vivo*, as well as the effects of various modulators of the enzymes in the hexosamine pathway.

The methods for analyzing glucosamine 6-phosphate and UDP-N-acetylglucosamine are based on the color reaction of N-acetylglucosamine with *p*-dimethylaminobenzaldehyde (Ehrlich's reagent) in dilute alkali solution (W.J. Morgan and L.A. Elson (1934) Biochem J. 28, 988-995). Modifications of this original procedure using a borate buffer for a more stable pH increased the yield by 2 fold and reduced the time of color development but also required high heat and ml quantities of reagents (J.L. Reissig, J.L. Strominger, and L.F. LeLoir ( 1955) JBC 217, 959-966). Acetylation of glucose 6-phosphate using a dilute acetic anhydride acetone solution followed by a borate alkali solution enabled quantitative conversion of glucosamine into N-acetylglucosamine but also required ml volumes and heating in boiling water (G.A. Levvy and A. McAllan (1959) Biochem J 73, 127-132). This method is capable of measuring glucosamine 6-phosphate in various tissues but requires the heating step in capped tubes (T.C. Richards and O. Greengard (1973) Biochemica et Biophysica Acta 304, 842-850). Others have measured GFAT activity in smaller quantities but the method requires perchloric acid to stop the reaction, neutralization with potassium hydroxide, centrifugation, and transfer of the supernatant for analysis (G.L. McKnight, S.L. Mudri, S.L. Mathewest, R.R. Traxinger, S. Marshall, P.O. Sheppard, and P.J. O'Hara (1992), JBC 267, 25204-25212). A spectrophotometric method for quantitation of UDP-N-acetylglucosamine has not been described. The current methods require purification of the sample and analysis by HPLC/MS (K.A. Robinson, M.L. Weinstein, G.E. Lindenmayer, and M.G. Buse (1995) Diabetes 44 1438-1446).

WO 93/21330 (Zymogenetics) discloses DNA molecules encoding human glutamine:fructose-6-phosphate amidotransferase. The DNA molecules are used in methods to screen for glutamine:fructose-6-phosphate amidotransferase antagonists. The DNA molecules encoding human glutamine:fructose-6-phosphate amidotransferase are expressed in suitable host cells and recombinant glutamine:fructose-6-phosphate amidotransferase is produced. A test substance is exposed to the recombinant human glutamine:fructose-6-phosphate amidotransferase in the presence of fructose-6-phosphate and glutamine. A reduction in activity of the glutamine:fructose-6-phosphate amidotransferase in comparison to the activity in the absence of the test substance indicates a compound that inhibits human glutamine:fructose-6-phosphate amidotransferase.

United States patent no. 5,876,713 (*Nishi et al*.) discloses glutamine:fructose-6-phosphate amidotransferase; a DNA coding for the protein; a recombinant vector; a transformant; a method for producing the protein; a pharmaceutical composition comprising the protein; and an antibody against the protein or its partial peptide. The protein and the DNA are used as a prophylactic or therapeutic agent for hypoglycemia. The antibody is used in the assay of the protein and the protein is used as a reagent for the screening for candidate medical compounds.

Figure 1 shows that the production of glucosamine 6-phosphate is dependent upon the amount of GFAT enzyme added to the reaction mixture. Figure 1A is a graph illustrating the dependence of glucosamine 6-phosphate production upon the amount of GFAT-*alpha* enzyme. Figure 1B is a graph illustrating the dependence of glucosamine 6-phosphate production upon the amount of GFAT-*beta* enzyme.

Figure 2 is a graph illustrating that the levels of glucosamine 6-phosphate produced are dependent on the time of incubation with the GFAT enzyme.

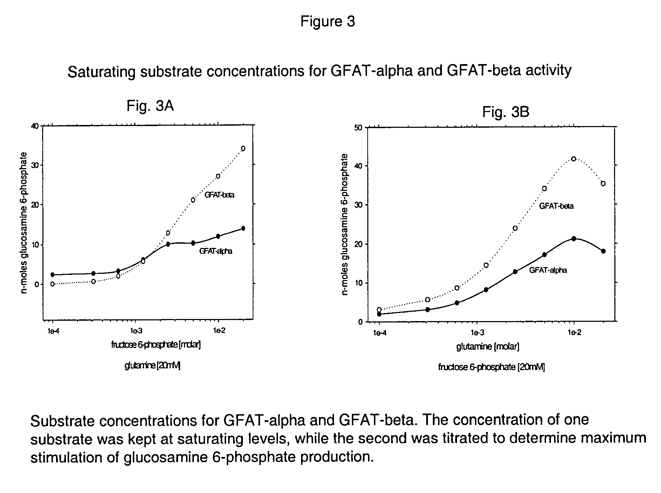

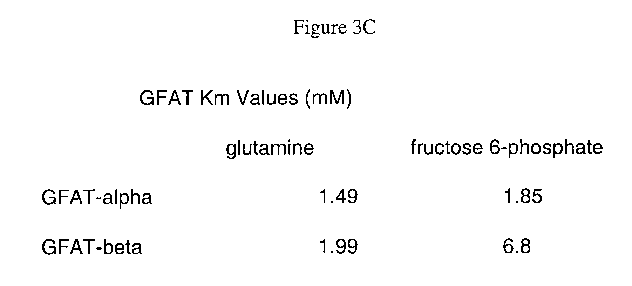

Figure 3 illustrates the effects of varying substrate concentrations, glutamine and fructose 6-phosphate, on the levels of glucosamine 6-phosphate produced by GFAT-*alpha* and GFAT-*beta*. Figure 3A is a graph illustrating the results of incubating GFAT with 10µl glutamine 200mM + 10µl fructose 6-phosphate 3.125, 6.25, 12.5, 25, 50, 100, and 200mM under standard incubation conditions at 37°C for 60 minutes. Figure 3B is a graph illustrating the results of incubating GFAT with 10µl fructose 6-phosphate 200mM + 10µl glutamine 3.125, 6.25, 12.5, 25, 50, 100, and 200mM under standard incubation conditions at 37°C for 60 minutes.

Figure 3C is a table that illustrates the Km values of the substrates for the GFAT-*alpha* and GFAT-*beta* enzymes calculated from the data generated in Figure 3A and 3B.

Figure 4 illustrates the determination of the optical density at 585nm for known quantities of D-glucosamine 6-phosphate made in 50% DMSO at a concentration of 40mM when added to the standard incubation mixture in the absence of active GFAT enzymes.

Figure 5 is a table that summarizes the results of testing known GFAT inhibitors for their effects on GFAT-*alpha* and GFAT-*beta* enzymes.

Figure 6 is a graph that illustrates the effects of acetylating glucosamine 6-phosphate with different concentrations of acetic anhydride.

Figure 7 is a bar graph that illustrates the effects of time and temperature on glucosamine 6-phosphate acetylation in a microtiter plate determined by the optical density (OD) at 585nm in the reaction mixture.

Figure 8 is a bar graph illustrating a comparison of N-acetylglucosamine produced by different methods.

Figure 9 is a graph that illustrates the effect of time and temperature on the hydrolysis of UDP-N-acetylglucosamine to N-acetylglucosamine.

Figure 10 is a bar graph that illustrates a comparison of the determination of UDP-N-acetylglucosamine levels obtained with the HPLC/MS method and the acid hydrolysis method in extracts of COS cells transfected with GFAT-*beta*.

The present invention relates to a method for measuring the activity of glutamine:fructose 6-phosphate amidotransferase, which comprises the steps of
(a) forming a mixture of glutamine:fructose 6-phosphate amidotransferase, fructose 6-phosphate, and glutamine;
(b) incubating the mixture in (a) under physiological conditions for a time sufficient to allow the formation of glucosamine 6-phosphate;
(c) acetylating the glucosamine 6-phosphate in (b) to form N-acetylglucosamine 6-phosphate;
(d) reacting the N-acetylglucosamine 6-phosphate in (c) with Ehrlich's reagent; and
(e) measuring the amount of N-acetylglucosamine 6-phosphate present in (d) by determining the optical density at 500-610nm of the mixture in (d) and comparing the amount of N-acetylglucosamine 6-phosphate in (d) with a control sample of N-acetylglucosamine 6-phosphate and a non-incubated control sample of glutamine:fructose 6-phosphate amidotransferase, or an incubated control sample of glutamine: fructose 6-phosphate amidotransferase without glutamine or without fructose 6-phosphate.

Preferably, the glutamine:fructose 6-phosphate amidotransferase is glutamine:fructose 6-phosphate amidotransferase-human *alpha* form having a Km of 1.49mM for glutamine and a Km of 1.85mM for fructose 6-phosphate or glutamine:fructose 6-phosphate amidotransferase-human *beta* form having a Km of 1.99mM for glutamine and a Km of 6.8mM for fructose 6-phosphate. The glutamine:fructose 6-phosphate amidotransferase may or may not be transfected into cells. Preferably, the mixture in (a) comprises glutamine:fructose 6-phosphate amidotransferase (0.25-25µl), glutamine (2-40mM), fructose 6-phosphate (2-40mM), phosphate buffered saline pH 7.4, ethylenediaminetetraacetic acid (5mM), and dithiothreitol (1mM), the incubation step in (b) is carried out at 37°C for a time period of 5 minutes to 5 hours, and the acetylation step in (c) is carried out with acetic anhydride 0.09375% in acetone followed by addition of potassium tetraborate 62.5mM at 80°C for 25 minutes. The Ehrlich's reagent in (d) preferably comprises 2g p-dimethylaminobenzaldehyde, 0.3ml water, 2.2ml concentrated hydrochloric acid, and 17.4ml acetic acid, which is diluted 1:2 in acetic acid. In a preferred embodiment, the mixture in (a) is formed in a microtiter plate and further comprises the step of adding a control sample of N-acetylglucosamine 6-phosphate and a non-incubated control sample of glutamine: fructose 6-phosphate amidotransferase, or an incubated control sample of glutamine:fructose 6-phosphate amidotransferase without glutamine or without fructose 6-phosphate to the microtiter plate, after the incubation step in (b).

The present invention also relates to a method for measuring the inhibitory activity of a test compound on glutamine:fructose 6-phosphate amidotransferase, which comprises the steps of
(a) forming a mixture of a test compound, glutamine:fructose 6-phosphate amidotransferase, fructose 6-phosphate, and glutamine;
(b) incubating the mixture in (a) under physiological conditions for a time sufficient to allow the test compound to inhibit glutamine:fructose 6-phosphate amidotransferase from forming glucosamine 6-phosphate;
(c) acetylating the glucosamine 6-phosphate in (b) to form N-acetylglucosamine 6-phosphate;
(d) reacting the N-acetylglucosamine 6-phosphate in (c) with Ehrlich's reagent; and
(e) measuring the amount of N-acetylglucosamine 6-phosphate in (d) by determining the optical density at 500-610nm of the mixture in (d) and comparing the amount of N-acetylglucosamine 6-phosphate in (d) with a control sample of glutamine:fructose 6-phosphate amidotransferase not containing the test compound and a control sample of N-acetylglucosamine 6-phosphate, thereby determining the inhibitory activity of the test compound.

Preferably, the glutamine:fructose 6-phosphate amidotransferase is glutamine:fructose 6-phosphate amidotransferase-human *alpha* form having a Km of 1.49mM for glutamine and a Km of 1.85mM for fructose 6-phosphate or glutamine:fructose 6-phosphate amidotransferase-human *beta* form having a Km of 1.99mM for glutamine and a Km of 6.8mM for fructose 6-phosphate. The glutamine:fructose 6-phosphate amidotransferase may or may not be transfected into cells. Preferably, the mixture in (a) comprises test compound, glutamine:fructose 6-phosphate amidotransferase (0.25-25µl), glutamine (10mM), fructose 6-phosphate (10mM), phosphate buffered saline pH 7.4, ethylenediaminetetraacetic acid (5mM), and dithiothreitol (1mM), the incubation step in (b) is carried out at 37°C for 1 hour, and the acetylation step in (c) is carried out with acetic anhydride 0.09375% in acetone followed by addition of potassium tetraborate 62.5mM at 80°C for 25 minutes. The Ehrlich's reagent in (d) preferably comprises 2g p-dimethylaminobenzaldehyde, 0.3ml water, 2.2ml concentrated hydrochloric acid, and 17.4ml acetic acid, which is diluted 1:2 in acetic acid. In a preferred embodiment, the mixture in (a) is formed in a microtiter plate and further comprises the step of adding a control sample of N-acetylglucosamine 6-phosphate and a non-incubated control sample of glutamine:fructose 6-phosphate amidotransferase, or an incubated control sample of glutamine:fructose 6-phosphate amidotransferase without glutamine or without fructose 6-phosphate to the microtiter plate, after the incubation step in (b).

The present invention further relates to a method for measuring the formation of uridine-diphosphate-N-acetylglucosamine in extracts from human and animal tissue or cells in tissue cultures, which comprises the steps of (a) hydrolyzing an extract from human or animal tissue or cells in tissue culture to convert uridine-diphosphate-N-acetylglucosamine to N-acetylglucosamine; (b) reacting the N-acetylglucosamine in (a) with Ehrlich's reagent; and (c) measuring the amount of N-acetylglucosamine present in (b) by determining the optical density at 500-610nm of the mixture in (b) and comparing the amount of N-acetylglucosamine in (b) with a control sample of N-acetylglucosamine, thereby determining the formation of uridine-diphosphate-N-acetylglucosamine.

Preferably, the extracts are from human tissue or cells. Preferably, the hydrolyzing step in (a) is carried out with hydrochloric acid 0.1N, followed by addition of potassium tetraborate 62.5mM at 80°C for 10 minutes and the Ehrlich's reagent in (b) comprises 2g p-dimethylaminobenzaldehyde, 0.3ml water, 2.2ml concentrated hydrochloric acid, and 17.4ml acetic acid, which is diluted 1:2 in acetic acid. In a preferred embodiment, the hydrolyzing step in (a) is carried out in a microfuge tube and the measuring step in (c) is carried out in a microtiter plate.

The present invention still further relates to a method for measuring the inhibitory activity of a test compound on uridine-diphosphate-N-acetylglucosamine formation in cells, which comprises the steps of (a) incubating a test compound with cells in tissue culture under physiological conditions for a time sufficient to allow the test compound to inhibit glutamine:fructose 6-phosphate amidotransferase and reduce cellular levels of uridine-diphosphate-N-acetylglucosamine; (b) extracting the uridine-diphosphate-N-acetylglucosamine in (a); (c) hydrolyzing the uridine-diphosphate-N-acetylglucosamine in (b) to form N-acetylglucosamine; (d) reacting the N-acetylglucosamine in (c) with Ehrlich's reagent; and (e) measuring the amount of N-acetylglucosamine in (d) by determining the optical density at 500-610nm of the mixture in (d) and comparing the amount of N-acetylglucosamine in (d) with the amount in cells in tissue culture not incubated with the test compound and a control sample of N-acetylglucosamine, thereby determining the inhibitory activity of the test compound.

Preferably, the cells in (a) are selected from the group consisting of type 293 human kidney cells, type 293 human kidney cells transfected with GFAT-*beta*, or COS monkey kidney cells, COS monkey kidney cells transfected with GFAT-*beta*, mammalian cells, yeast cells, and bacterial cells. Preferably, the hydrolyzing step in (c) is carried out with hydrochloric acid 0.1N, followed by addition of potassium tetraborate 62.5mM at 80°C for 10 minutes and the Ehrlich's reagent in (d) comprises 2g p-dimethylaminobenzaldehyde, 0.3ml water, 2.2ml concentrated hydrochloric acid, and 17.4ml acetic acid, which is diluted 1:2 in acetic acid.

The present invention still further relates to a method for measuring the inhibitory activity of a test compound on uridine-diphosphate-N-acetylglucosamine formation in a human or an animal, which comprises the steps of
(a) dosing a human or an animal with a test compound under physiological conditions for a time sufficient to allow the test compound to inhibit glutamine:fructose 6-phosphate amidotransferase from forming uridine-diphosphate-N-acetylglucosamine;
(b) removing tissue from the human or animal in (a) and extracting the uridine-diphosphate-N-acetylglucosamine from the tissue;
(c) hydrolyzing the uridine-diphosphate-N-acetylglucosamine in (b) to form N-acetylglucosamine;
(d) reacting the N-acetylglucosamine in (c) with Ehrlich's reagent; and
(e) measuring the amount of N-acetylglucosamine in (d) by determining the optical density at 500-610nm of the mixture in (d) and comparing the amount of N-acetylglucosamine in (d) with the amount in a human or an animal not dosed with the test compound and a control sample of N-acetylglucosamine, thereby determining the inhibitory activity of the test compound.

Preferably, the dosing in (a) is carried out orally or intravenously 1 to 2 times per day over a period of 1 day to 2 weeks and the animal in (a) is a human, mouse, or rat. Preferably, the hydrolyzing step in (c) is carried out with hydrochloric acid 0.1N, followed by addition of potassium tetraborate 62.5mM at 80°C for 10 minutes.

The present invention relates to rapid spectrophotometric methods for high throughput microtiter plate analysis of glucosamine 6-phosphate levels. The methods also enable the simple measurement and quantitation of UDP-GlcNAc from a variety of sources. The spectrophotometric methods described are preferably performed in a microtiter plate and are based on the color reaction of N-acetylglucosamine with *p*-dimethylaminobenzaldehyde (Ehrlich's reagent) in dilute alkali solution. The spectrophotometric methods do not require the many purification steps needed for HPLC/MS methods and are capable of assaying many samples at a time. The methods include the hydrolysis of UDP-N-acetylglucosamine to N-acetylglucosamine, which is then measured by the spectrophotometric method for N-acetylglucosamine.

As used herein, the term "physiological conditions" refers to concentration, temperature, pH, ionic strength, viscosity, time, and various biochemical parameters which are compatible with a viable organism, and/or which typically exist intracellularly in a viable cultured animal cell or mammalian cell. Suitable reaction conditions for *in vitro* enzyme reactions are generally physiological conditions. The incubation of the mixture of glutamine:fructose 6-phosphate amidotransferase, fructose 6-phosphate, and glutamine is generally carried out under physiological conditions for a time sufficient to allow the formation of glucosamine 6-phosphate. For the measurement of glucosamine 6-phosphate, the preferred physiological conditions comprise NaCl 150mM at pH 7.4 and 37°C. For the measurement of uridine-diphosphate-N-acetylglucosamine, the cells are preferably grown in Delbecco's Modified Eagles Medium (DMEM) at pH 7.4 with 10% fetal bovine serum. Particular aqueous conditions may be selected by the practitioner according to conventional methods with the optional addition of divalent cation(s) and/or metal chelators and/or non-ionic detergents and/or membrane fractions and/or anti-foam agents.

GFAT inhibitors reduce or inhibit the production of glucosamine 6-phosphate and ultimately the production of UDP-N-acetylglucosamine. The *in vitro* method using glutamine:fructose 6-phosphate amidotransferase only permits the measurement of glucosamine 6-phosphate. Another consideration in identifying a GFAT inhibitor of potential use in treatment of patients is whether the test compound enters cells *in vivo.* Under physiological conditions in cells, biological agents, e.g., enzymes, are produced in connection with the hexosamine biosynthetic pathway, which are involved in the production of UDP-GlcNAc. Compounds that are found to inhibit GFAT enzyme activity in cells can be tested in animals to determine if they enter the tissues, inhibit GFAT activity, and inhibit production of UDP-GlcNAc.

For measurement of N-acetylglucosamine 6-phosphate, the GFAT enzyme reaction and analysis of the product are preferably carried out directly in a microtiter plate. The preferred reaction volume of 100µl consists of the substrates glutamine (10mM) and fructose 6-phosphate (10mM), phosphate buffered saline (PBS) pH 7.4, ethylenediaminetetraacetic acid (EDTA) (5mM), dithiothreitol (DTT) (1mM), and the GFAT enzyme preparation or tissue extract (0.25-50µl). The incubation mixture is added to the plate, the plate sealed, and placed floating on a 37°C water bath for 1 hour. Additional incubation mixture is kept on ice to add to standard and control samples. After incubation, non-incubated control and glucosamine 6-phosphate standards of 2.5 to 30 nmoles along with the cold incubation mixture are added to the appropriate wells in the plate. The glucosamine 6-phosphate produced in the incubation mixtures is acetylated by first adding acetic anhydride 1.5% in acetone (10µl), followed by potassium tetraborate 200mM (50µl). The plate is sealed, shaken for 2 minutes on a microshaker, and placed on an 80°C water bath for 25 minutes. The plate is cooled by placing it on ice for 5 minutes and then Ehrlich's reagent is added (130µl) (2g Ehrlich's reagent + 0.3ml water + 2.2ml concentrated hydrochloric acid + 17.4ml acetic acid which is diluted 1:2 in acetic acid before use). The plate is then placed on a 37°C water bath for 20 minutes and the OD determined at 500-610nm, preferably 585nm.

For measurement of UDP-N-acetylglucosamine in extracts from human and animal tissue or cells in tissue cultures, the extract sample is first hydrolyzed in 0.1N HCl at 80°C for 10 minutes to produce N-acetylglucosamine. The sample is then cooled on ice for 5 minutes and neutralized with 0.1N KOH. Potassium tetraborate (62.5mM [final]) is then added and the sample incubated at 80°C for 25 minutes and then cooled on ice for 5 minutes. Ehrlich's reagent is then added and the sample is maintained for 20 minutes at 37°C and then the OD is determined at 500-610nm, preferably 585nm.

| **Stock Reagents for the Microtiter Plate Assay for Glucosamine 6-Phosphate** | |
|---|---|
| Glutamine | 100mM |
| Fructose 6-phosphate | 100mM |
| PBS | 10X |
| EDTA | 50mM |
| DTT | 10mM |
| Acetic Anhydride | 1.5%/Acetone |
| Potassium Tetraborate | 200mM |
| p-Dimethylaminobenzaldehyde (Ehrlich's Reagent) | 2g p-Dimethylaminobenzaldehyde/0.3ml water 2.2ml conc. HCl/17.4ml Acetic Acid diluted 1:2 in Acetic Acid before use |
| N-Acetylglucosamine for standards | |
| Enzyme Preparation or Tissue Extract | |

| **Incubation Amounts for the Microtiter Plate Assay for Glucosamine 6-Phosphate** | |
|---|---|
| Glutamine 100mM | 10ul |
| Fructose 6-phosphate 100mM | 10ul |
| PBS 10X | 10µl |
| EDTA 50mM | 10µl |
| DTT 10mM | 10µl |
| +/- Inhibitor | 10µl |
| Enzyme Preparation or Tissue Extract | 0.25-50µl |
| Water | to 100µl |
| Acetic Anhydride 1.5%/acetone | 10µl |
| Potassium Tetraborate 200mM | 50µl |
| Ehrlich's reagent | 130µl |

The reaction mixture is made including enough for standard curve samples and kept on ice. The reaction is started by adding the incubation reagents to the microtiter plate, sealing the plate, and placing the plate in a water bath at 37°C for 1 hr. Standard curve samples are added to the plate, 2.5 to 30 nmoles/well/10µl, and the cold mix added to control and standard curve samples. Glucosamine 6-phosphate is acetylated with acetic anhydride 1.5%/acetone 10µl + potassium tetraborate 200mM 50µl, by sealing the plate, shaking for 2 minutes and placing on an 80°C water bath for 25 minutes, then cooling on ice for 5 minutes. Diluted Ehrlich's reagent 130µl is added and the plate placed on a 37°C water bath for 20 minutes. The OD is determined at 585nm.

| **Stock Reagents for the Spectrophotometric Assay for Measuring UDP-N-Acetylglucosamine** | |
|---|---|
| HCl | 1N |
| KOH | 1N |
| Potassium Tetraborate | 200mM |
| p-Dimethylaminobenzaldehyde (Ehrlich's Reagent) | 2g p-Dimethylaminobenzaldehyde/0.3ml water 2.2ml conc. HCl/17.4ml Acetic Acid diluted 1:2 in Acetic Acid before use |
| Acetonitrile | |
| N-Acetylglucosamine for standards | |
| Enzyme Preparation or Tissue Extract | |

| **Incubation Mixture for the Spectrophotometric Assay for Measuring UDP-N-Acetylglucosamine** | |
|---|---|
| +/- Inhibitor | 10µl |
| Acetonitrile (if needed) | 50µl |
| HCl 1N | 10µl |
| KOH 1N | 10µl |
| Potassium Tetraborate 200mM | 50µl |
| Ehrlich's reagent | 150µl |
| Cells or Tissue Extract | 50-100µl |
| Water | to 100µl |

The reaction mixture is made as described above for the glucosamine 6-phosphate assay except the reaction is preferably started in a microfuge tube. If the sample contains too much protein, acetonitrile is added to first precipitate the protein to clarify the solution. The sample is then hydrolyzed in HCl 0.1N at 80°C for 10 minutes, cooled on ice, and centrifuged 5 minutes to remove debris if there is high protein content. The sample is neutralized with KOH 0.1N. K₂B₄0₇ (59mM [final]) is added and the sample incubated at 80°C for 25 minutes and then cooled on ice for 5 minutes. Ehrlich's reagent is added for 20 minutes at 37°C, the samples centrifuged for 2 minutes, and 200µl of the supernatant is added to a microtiter plate. The OD is determined at 585nm.

Throughout this disclosure, applicant will suggest various theories or mechanisms by which applicant believes the present methods function. While applicant may offer various mechanisms to explain the present invention, applicant does not wish to be bound by theory. These theories are suggested to better understand the present invention but are not intended to limit the effective scope of the claims.

Throughout this application, various publications have been referenced. The disclosures in these publications are incorporated herein by reference in order to more fully describe the state of the art.

The present invention is further illustrated by the following examples that are presented for purposes of demonstrating, but not limiting, the preparation of the compounds and compositions of this invention.

### Examples

### Example 1: Measurement of Glucosamine 6-Phosphate

### Enzyme preparation.

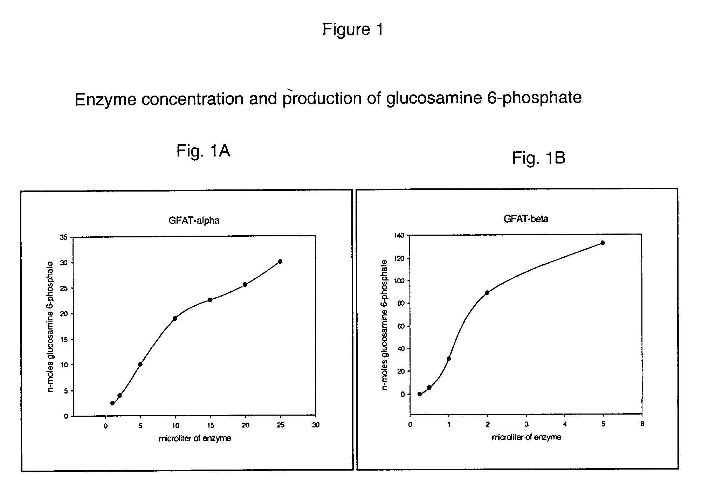

GFAT-*alpha* (GenBank Accession No. M90516) and GFAT-*beta* (Accession No. AB016789) are cloned into pET12 and pEF-BOS C vectors (New England BioLabs) for transfection into SF9 and Hi5 insect cells, COS, type 293 cells, and E.coli. Endogenous activity for each cell line was also measured in non-transfected cells. The enzymes are expressed in each cell line by growing in large 24x24 cm plates and harvesting 20 hours after transfection. The media is removed and the plates are washed in 15ml PBS. 1.5ml of a lysis buffer (PBS 1X, KCl 50mM, EDTA 10mM, with protease inhibitors leupeptin 10ug/ml, PMSF 20ug/ml, A-protinin 30ug/ml, and pepstatin 10ug/ml) is added to each plate and the cells are removed using a large plastic cell scraper. This results in a final volume of about 3 ml. The cell #/lysis volume is about 4x10⁷ cell/ml. The suspension is sonicated on ice (Branson sonicator setting 2.5 to 3) for 15 seconds using a microtip. The sonicated cell extracts are stored in microfuge tubes at -80°C. Before use, the extracts are centrifuged at 15000 xg for 5 minutes at 4°C. Each enzyme preparation is titrated to determine the volume that gives between 20 to 30 nmoles of glucosamine 6-phosphate in a reaction mixture in a 60-minute incubation at 37°C (Figures 1A and 1B).

### Stimulation of GFAT activity.

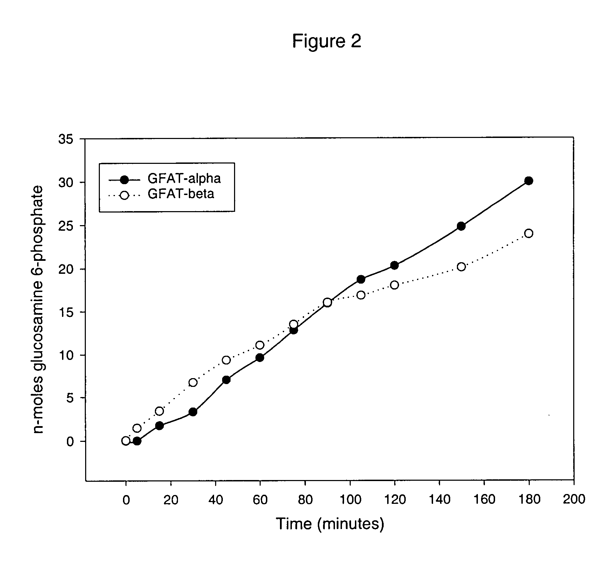

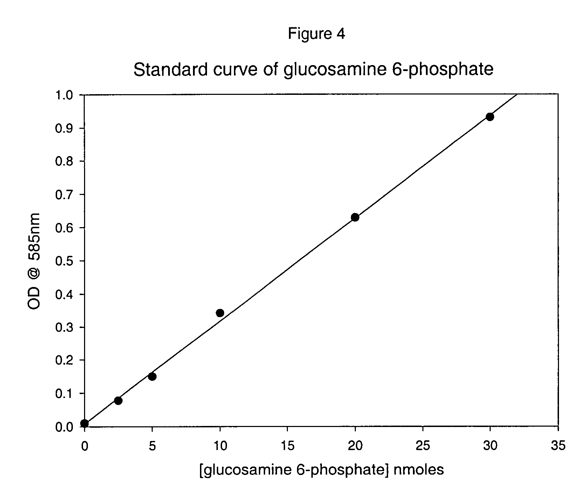

The enzyme reaction was found to be linear over time (Figure 2) in the presence of saturating substrate levels. For most testing, a 60-minute incubation at 37°C is carried out in 96 well microtiter plates. To give the best heat transfer the plates are sealed with a Costar plate sealer and floated on a 37°C water bath. Air bubbles are removed from the bottom of the plate to ensure proper heat transfer to all wells. The 100µl reaction volume consists of glutamine 10mM, fructose 6-phosphate 10mM, PBS 1X, EDTA 5mM, DTT 1mM, and the enzyme preparation. A mixture of the incubation reagents is kept on ice and the enzyme is added to the mixture just before addition to the plate. Additional incubation mixture is kept on ice to add to standard and control samples. The reaction did not take place at 4°C. If test agents are to be used, 10µl is added to the appropriate wells and the vehicle is added to the control wells before addition of the incubation mixture. 15 wells are left blank for addition of control and standard glucosamine 6-phosphate samples. After the incubation, 10µl of the standards made up in the appropriate vehicle (DMSO) are added to the appropriate wells. A concentration range of 2.5 to 30 nmoles is in the linear portion of the curve and covers the quantity of glucosamine 6-phosphate produced under the incubation conditions (Figure 4). The cold mixture is added to the standard sample wells and the glucosamine 6-phosphate is then measured.

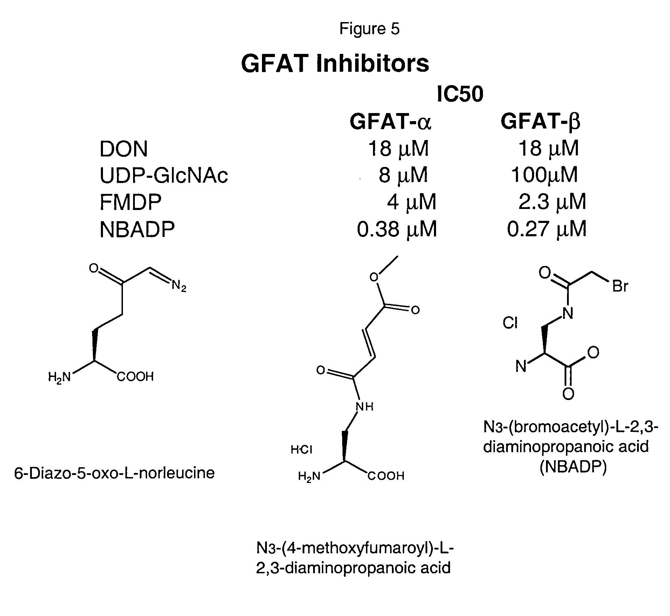

The activity of the GFAT-*alpha* and GFAT-*beta* enzymes expressed in SF9 insect cells were characterized with known inhibitors of GFAT (Figure 5). DON (6-diazo-5-oxo-L-norleucine) inhibited both enzymes with an IC50 of 18µM. UDP-N-acetylglucosamine inhibited GFAT-*alpha* with an IC50 of 8µM, but more than 12 times as much UDP-N-acetylglucosamine was required to inhibit GFAT-*beta* having an IC50 of 100µM. The glucosamine analog FMDP (N3-(-4-methoxyfumaroyl)-L-2,3-diaminopropanoic acid) inhibited GFAT-*alpha* with an IC50 of 4µM and GFAT-*beta* with an IC50 of 2.3µM. N3-(bromoacetyl)-L-2,3-diaminopropanoic acid inhibited GFAT-*alpha*, IC50 0.38µM, and GFAT-*beta*, IC50 0.27µM. The inhibition with UDP-N-acetylglucosamine is thus a discriminator between GFAT-*alpha* and GFAT-*beta*, and provides information about the functional roles of the two enzymes.

### Measurement of glucosamine 6-phosphate

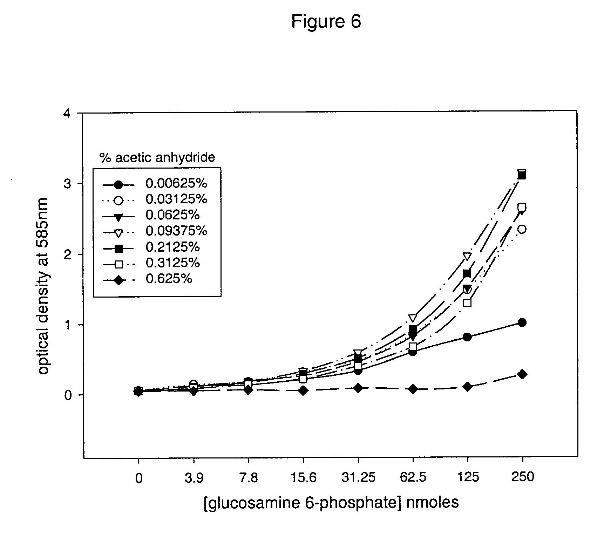

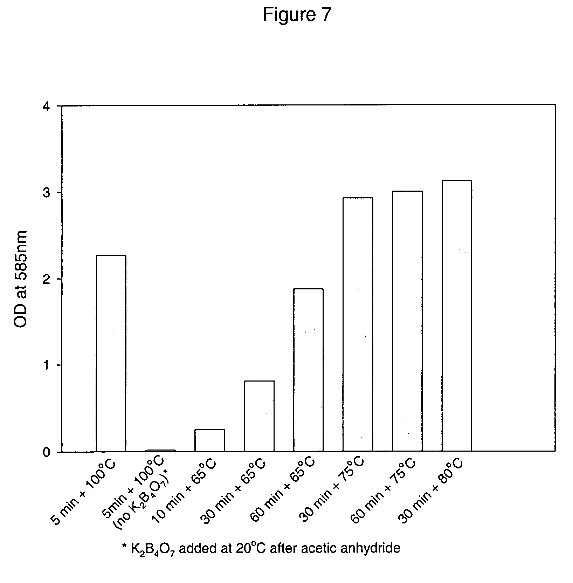

The reaction is started by adding the incubation mixture to the appropriate wells in a microtiter plate and placing the plate on a 37°C water bath. After the appropriate incubation time, the reaction is stopped by adding 10µl of 1.5% acetic anhydride (final 0.09375%) followed by 50µl of potassium tetraborate 200mM (final 62.5mM). The plate is sealed and placed on a micro-shaker for 2 minutes. The plate is then floated on an 80°C water bath for 30 minutes taking care to remove air bubbles under the plate. 0.09375% acetic anhydride (out of a range of 0.00625% to 0.625%) at 80°C (out of a range of 20° to 80°C) was found to be the best concentration for acetylation of glucosamine 6-phosphate (Figure 6), and 30 minutes (out of a range of 10 to 30 minutes) at 80°C (out of a range of 65°C to 80°C) to be a good incubation condition (Figure 7). After the acetylation at 80°C, the plate is cooled on ice for 5 minutes. The acetylated glucosamine is measured by the Morgan and Elson color reaction (Morgan. W.T.J. & Elson, L.A. (1934).Biochem. J. 28, 988). 130µl of Ehrlich's reagent (2g p-dimethylaminobenzaldehyde + 0.3ml water + 2.2ml conc. HCl + 17.4ml acetic acid which is diluted 1:2 in acetic acid before use) is added to each well and incubated at 37°C by floating on a water bath for 20 minutes. The OD is determined at 585nm and the quantity of glucosamine 6-phosphate produced is calculated from the standard curve using a Softmax-Pro program to interpolate the ODs from the standard curve to give the nmoles produced.

### Example 2: Measurement of UDP-N-Acetylglucosamine in Cells and Tissue

### Tissue extract.

Tissue to be assayed is kept frozen at -80°C and kept on dry ice during the preparation and weighing process. The tissue is pulverized to a fine powder by rapping the tissue in heavy foil, placing it on top of a metal block in a bed of dry ice, and rapidly hammering it until it is a fine powder. It is then placed in a tarred microfuge tube and kept on dry ice until weighed. The tissue is extracted in chloroform:water (1mg +2µl chloroform + 2µl water). The suspension is sonicated for about 5 seconds on ice (Branson sonicator setting 3 to 5 with micro-tip) to breakup any clumps. The extract can then be stored at -80°C. Before analysis, the tissue extract is then thawed and centrifuged at 15000xg for 20 minutes at 4°C.

### Cell extract:

Cells in culture are incubated in 35mm culture dishes with and without a GFAT inhibitor and treated with glucose to produce UDP-N-acetylglucosamine. Cells are normally grown to about 80% confluency in DMEM-glucose 4.5mM + 10% fetal bovine serum. The media is then changed to DMEM (no glucose) + 10% fetal bovine serum for 12 to 16 hours. If a GFAT inhibitor is present, it is added for 30 minutes, and followed by addition of glucose 25mM for time periods of 1 hour to 24 hours. The media is removed, cells washed 1X in PBS, and extracted in 200µl of chloroform:water (1:1). The suspension is sonicated for about 5 seconds on ice to breakup any clumps. The extract can be stored at -80°C. Before analysis, the tissue extract is then thawed and centrifuged at 15000xg for 20 minutes at 4°C.

### Analysis of UDP-N-Acetylglucosamine in tissue extracts.

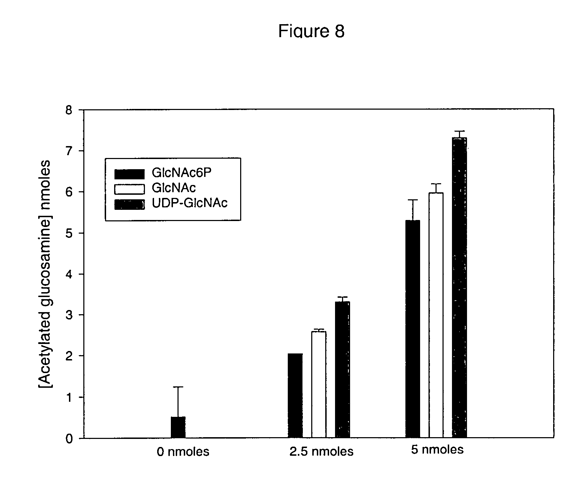

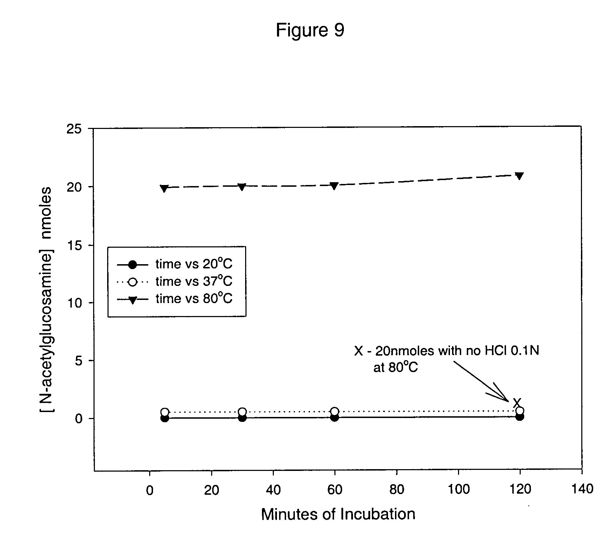

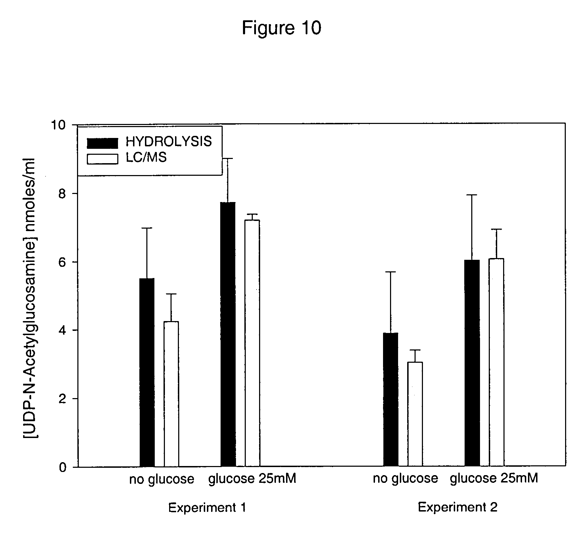

50µl of tissue extract (25mg starting tissue) is placed in a microfuge tube and 50µl of acetonitrile is added. This is needed to precipitate excess protein in the sample and help clarify the sample. To hydrolyze the UDP-N-acetylglucosamine in the sample, 10µl of HCl 1N is added and the sample vortexed. This mixture is heated at 80°C for 20 minutes. The sample is then centrifuged 15000xg at room temperature for 15 seconds to precipitate any condensation formed. The sample is neutralized with the addition of 10µl KOH 1N. The resulting product, N-acetylglucosamine, is measured by the Morgan and Elson color reaction. 50µl of potassium tetraborate 200mM is added and the sample heated at 80°C for 25 minutes followed by cooling on ice for 5 minutes. 150µl of Ehrlich's reagent is added and mixed. After 20 minutes at 37°C, the sample is centrifuged 15000xg at room temperature for 2 minutes. 200µl of the supernatant is added to a 96 well plate and the OD determined at 585nm. The quantity of N-acetylglucosamine is calculated from standard samples of N-acetylglucosamine using a Softmax-Pro program. The standard curve samples are prepared from N-acetylglucosamine, 2.5 to 30 nmoles/well, in a similar manner to the tissue samples. Comparison experiments were performed to confirm the analysis of N-acetylglucosamine produced by three different methods. The experiments demonstrated that: 1) glucosamine 6-phosphate acetylated with acetic anhydride + potassium tetraborate, 2) N-acetylglucosamine treated with potassium tetraborate, and 3) UDP-N-acetylglucosamine hydrolyzed with HCl 0.1N followed by treatment with potassium tetraborate all gave similar results (Figure 8). The data also demonstrates that the UDP-N-acetylglucosamine is completely hydrolyzed to N-acetylglucosamine with HCl 0.1N after 20 minutes at 80°C. Studies on time (5 to 120 minutes) and temperature (20, 37, and 80°C) indicate that 5 minutes at 80°C is sufficient for complete hydrolysis of UDP-N-acetylglucosamine to N-acetylglucosamine (Figure 9). These studies also indicate that UDP-N-acetylglucosamine treated with potassium tetraborate does not react with Ehrlich's reagent to produce a color. To verify that the hydrolysis method and the HPLC/MS method give comparable results extracts of COS cells were analyzed by the two methods. COS cells transfected with GFAT-*beta* grown in glucose free media for 16 hours were exposed to glucose 25mM for 5 hours. Glucose treatment results in an increase in cellular UDP-N-acetylglucosamine levels. The measured levels of no glucose and glucose 25mM stimulated cells were comparable between the two methods (Figure 10).

Figure 1 shows that the production of glucosamine 6-phosphate is dependent upon the amount of GFAT enzyme. Figure 1A is a graph illustrating the dependence of glucosamine 6-phosphate production upon the GFAT-*alpha* enzyme concentration. Figure 1B is a graph illustrating the dependence of glucosamine 6-phosphate production upon the GFAT-*beta* enzyme concentration. To an incubation mixture of 10µl glutamine 100mM, 10µl fructose 6-phosphate 100mM, 10µl PBS 10X, 10µl EDTA 50mM, 10µl DTT 10mM, and water to 100µl is added 1, 2, 5,10, 15, 20, and 25µl of GFAT-*alpha* cell extracts, or 0.25, 0.5, 1, 2, and 5µl of GFAT-*beta* cell extracts. The incubation is started by adding the incubation mixture to the appropriate wells of a 96 well microtiter plate, separate plates are used for each enzyme, and placing (floating) the plates on a 37°C water bath. After a 60-minute incubation, the plates are removed from the water bath and the standard curve samples are added to the appropriate wells of the plate. This is followed by the addition of 10µl of acetic anhydride 1.5% and 50µl potassium tetraborate 200mM to all the wells and shaking the plate for 2 minutes at room temperature on a microshaker. The plates are incubated at 80°C by placing the plates (floating) on an 80°C water bath for 30 minutes. The plates are cooled on ice for 5 minutes. 130µl of Ehrlich's reagent is added to all the wells for 20 minutes at 37°C and the OD determined for all of the wells at 585nm. The nmoles of glucosamine 6-phosphate in each well is determined for each concentration of enzyme. Production of glucosamine-6-phosphate is dependent on the amount of GFAT enzyme. Increasing amounts of GFAT-alpha expressed in SF9 insect cells and GFAT-beta expressed in Hi5 insect cells were incubated at 37°C for 60 minutes with saturating amounts of substrates.

The differentiation of the GFAT-*alpha* from the GFAT-*beta* can be determined by their sensitivity to UDP-N-acetylglucosamine. GFAT-*alpha* is inhibited by UDP-N-acetylglucosamine with an IC50 of 8µM. GFAT-*beta* is inhibited by UDP-N-acetylglucosamine with an IC50 of 100µM.

Figure 2 is a graph illustrating that GFAT activity is dependent on the time of incubation as expressed in Hi5 insect cells. To determine the GFAT activity over time extracts of GFAT-*alpha* and GFAT-*beta* expressed in Hi5 insect cells are incubated over a 180-minute period. The reaction is done in 96 well microtiter plates. The incubation mixtures are 1) 10µl glutamine 100mM, 10µl fructose 6-phosphate 100mM, 10µl PBS 10X, 10µl EDTA 50mM, 10µl DTT 10mM, 5µl GFAT-*alpha* Hi5 cell extract, and 45µl water, and 2) 10µl glutamine 100mM, 10µl fructose 6-phosphate 100mM, 10µl PBS 10X, 10µl EDTA 50mM, 10µl DTT 10mM, 0.25µl GFAT-*beta* Hi5 cell extract, and 49.75µl water. The reaction mixtures are kept on ice and the GFAT-*alpha* and GFAT-*beta* enzymes incubated in different plates. The reaction is started by adding 100µl of the incubation mixture to the plate starting with the 180 minute time with the 0 time last. The reaction is stopped by placing the plate on ice. The standard samples are added to the appropriate wells of the plate followed by addition of 10µl of acetic anhydride 1.5% in acetone and 50µl of potassium tetraborate 200mM. The plates are shaken for 2 minutes at room temperature and are incubated at 80°C for 30 minutes by floating on a water bath. The plates are then placed on ice for 5 minutes followed by addition of Ehrlich's reagent 130µl. After a 20-minute incubation at 37°C, the OD at 585nm is determined for all the wells on the plate and the quantity of N-acetyl glucosamine 6-phosphate determined. Similar results are obtained with GFAT enzymes prepared from different sources.

Figure 3 illustrates the effect of varying concentrations of the substrates glutamine and fructose 6-phosphate on the enzyme activity of GFAT-*alpha* and GFAT-*beta*. Figure 3A is a graph illustrating incubation with 10µl glutamine 200mM + 10µl fructose 6-phosphate 3.125, 6.25, 12.5, 25, 50, 100, and 200mM. Figure 3B is a graph illustrating incubation with 10µl fructose 6-phosphate 200mM + 10µl glutamine 3.125, 6.25, 12.5, 25, 50, 100, and 200mM. 10µl of GFAT-*alpha* and 5µl of GFAT-*beta* extracts from transfected SF9 insect cells are incubated with 1) 10µl glutamine 200mM + 10µl fructose 6-phosphate 3.125, 6.25, 12.5, 25, 50, 100, and 200mM, and 2) 10µl fructose 6-phosphate 200mM + 10µl glutamine 3.125, 6.25, 12.5, 25, 50, 100, and 200mM in an incubation media containing 10µl PBS 10X, 10µl EDTA 50mM, 10 µl DTT 10mM and water to 100µl. The plate is sealed with a Costar plate sealer and incubated for 60 minutes at 37°C by floating on a water bath. Additional incubation mixture is kept on ice to add to standard and control samples. After the incubation, standards along with the cold incubation mixture are added to the appropriate wells on the plate, followed by 10µl acetic anhydride and 50µl potassium tetraborate 200mM to all the wells. The plate is shaken for 2 minutes and incubated at 80°C by floating on a water bath. The plate is cooled on ice for 5 minutes followed by addition of Ehrlich's reagent to each well. After a 20-minute incubation at 37°C, the OD is determined for each well at 585nm. Figure 3 substrate concentrations for GFAT-alpha and GFAT-beta. The concentration of one substrate was kept at saturating levels, while the second substrate was titrated to determine the maximum stimulation of glucosamine 6-phosphate production.

Figure 3C is a table illustrating the Km values of the substrates for the GFAT-*alpha* and GFAT-*beta* enzymes calculated from the data generated in Figures 3A and 3B. A plot of the data using a Lineweaver-Burk plot (Lineweaver, H. and Burk, D (1934) J.Amer.Chem.Soc. 56, 658-666) gave the Km values reported. In the table, the Km of glutamine for GFAT-*alpha* and GFAT-*beta* are 1.49mM and 1.99mM, respectively. The Km of fructose 6-phosphate for GFAT-*beta* is 6.8mM and for GFAT-*alpha* is1.85mM. Comparison of Km values for the two substrates for GFAT-*alpha* and GFAT-*beta* expressed in insect cells

Figure 4 is a graph illustrating the standards of D-glucosamine 6-phosphate (Sigma-Aldrich Cat# G5509) made in 50% DMSO at a concentration of 40mM. Dilutions are made from this stock solution to give 0, 2.5, 5, 10, 20, and 30nmoles in a 10µl sample. The OD of the 0mM, DMSO vehicle alone, is subtracted from all of the determined values on the plate. 10µl of the standard samples are added to the appropriate wells on the plate, in duplicate wells. The plate is sealed with a Costar plate sealer and incubated for 60 minutes at 37°C by floating on a water bath. During the incubation the incubation mixture is kept on ice. The incubation mixture consists of 10µl glutamine 100mM, 10µl fructose 6-phosphate 100mM, 10µl PBS 10X, 10µl EDTA 50mM, 10µl DTT 10mM, 2µl of an enzyme extract of COS cells transfected with GFAT-*alpha*, and 48µl water. After the incubation 90µl of the cold incubation mixture is added. The reaction is stopped forthwith by the addition of 10µl acetic anhydride 1.5% followed by 50µl of potassium tetraborate 200mM to all the wells. The plate is shaken for 2 minutes and incubated for 30 minutes at 80°C by floating on a water bath. The plate is cooled on ice for 5 minutes followed by the addition of Ehrlich's reagent to all the wells. After a 20-minute incubation at 37°C, the OD is determined for all the wells at 585nm.

Figure 5 illustrates the testing of known inhibitors of GFAT for sensitivity against the GFAT-*alpha* and GFAT-*beta* enzymes. The incubation mixture consists of 10µl glutamine 100mM, 10µl fructose 6-phosphate 100mM, 10µl PBS 10X, 10µl EDTA 50mM, 10 µl DTT 10mM, 5µl GFAT-*alpha* or 0.25µl GFAT-*beta*, 10µl of the test compound in DMSO 50% or DMSO 50% alone, and water to bring the final volume to 100µl. The compounds are added to the appropriate wells of a 96 well microtiter plate followed by 90µl of the incubation media for GFAT-*alpha* and GFAT-*beta* to the appropriate wells. The plate is incubated at 37°C by placing the plate on a water bath for 60 minutes. The standard samples are then added to the plate followed by 10µl of acetic anhydride 1.5% and 50µl potassium tetraborate 200mM to all the wells. After shaking the plate for 2 minutes, the plate is incubated at 80°C for 30 minutes. The plate is cooled on ice for 5 minutes and 130µl Ehrlich's reagent is added for 20 minutes at 37°C to all the wells. The OD for each well is determined at 585nm and the amount of glucosamine 6-phosphate produced is calculated. The amount of inhibition with the compound is determined from the enzyme activity in the presence of DMSO alone. The inhibition is expressed as the IC50 or the concentration inhibiting 50% of maximum stimulation.

Figure 6 is a graph illustrating the acetylation of glucosamine 6-phosphate with different concentrations of acetic anhydride. The reaction is carried out in a 96 well microtiter plate. The incubation mixture consists of glucosamine 6-phosphate 1µmole in 100µl + 10µl of acetic anhydride made up in acetone at initial concentrations of 0.1% to 10% which gives final concentrations of 0.00625% to 0.625% + 50µl potassium tetraborate 200mM. The mixture is incubated at 80°C for 30 minutes by sealing the plate with a Costar plate sealer and floating the plate on an 80°C water bath. After the incubation, the plate is cooled on ice for 5 minutes. 130µl of Ehrlich's reagent is added to all the wells, the plate sealed, and the plate is incubated at 37°C for 20 minutes by floating on a 37°C water bath. The OD is determined for all the wells at 585nm and the data is expressed as the optical density produced at the different concentrations of acetic anhydride.

Figure 7 is a bar graph illustrating the optimum conditions for acetylation of glucosamine 6-phosphate in wells of a microtiter plate by varying times and temperatures. To permit the assay to be performed in a microtiter plate temperatures less than 100°C are necessary. The incubations are performed in microfuge tubes in this assay for ease of handling the different conditions. 1µmole of glucosamine 6-phosphate in 100µl water + 22.5µl acetic anhydride 1.5% in acetone + 50µl potassium tetraborate 200mM + 80µl water are incubated under the different conditions. After each incubation, the tube is cooled on ice for 5 minutes and 140µl of Ehrlich's reagent is added to each tube for 20 minutes at 37°C. The OD is determined at 585nm. The following incubation conditions are performed: 1) As a standard, a 5 minute incubation of the acetylation mixture is placed in a boiling water bath for 5 minutes. 2) A 5-minute incubation with acetic anhydride alone at 100°C followed by potassium tetraborate for 5 minutes at room temperature is done to demonstrate the necessity of the heating step with potassium tetraborate. 3) A 10-minute incubation at 65°C. 4) A 30-minute incubation at 65°C. 5) A 60-minute incubation at 65°. 6) A 30-minute incubation at 75°C. 7) A 60-minute incubation at 75°C. 8) A 30-minute incubation at 80°C. As the temperature increases, the OD increases indicating better sensitivity. As the time is increased, the OD increases indicating better sensitivity.

Figure 8 is a bar graph illustrating a comparison of N-acetylglucosamine produced by different methods. Standard solutions of glucosamine 6-phosphate (Sigma-Aldrich G5509), N-acetylglucosamine (Sigma-Aldrich A8625), and UDP-N-acetylglucosamine (Sigma-Aldrich U4375) are dissolved in water to a concentration of 4mM and diluted to 2.5 and 5nmoles/10µl. The standard solutions are treated in the following manner: 1) Glucosamine 6-phosphate is acetylated by adding 10µl of each solution to 90µl water, 10µl HCl 1N, 10µl KOH 1N, and 10µl acetic anhydride 1.5% followed by 50µl potassium tetraborate 200mM. The mixture is heated to 80°C in a water bath for 30 minutes and then cooled on ice for 5 minutes. 2) 10µl of the N-acetylglucosamine solutions are added to 90µl water, 10µl HCl 1N, 10µl KOH 1N, and 50µl potassium tetraborate 200mM. The mixture is heated at 80°C in a water bath for 30 minutes and then cooled on ice for 5 minutes. 3) 10µl of each standard solution of UDP-N-acetylglucosamine is added to 90µl water and 10µl HCl 1N. This mixture is hydrolyzed by heating in an 80°C water bath for 10 minutes and then centrifuged 15000 xg for 15 seconds. 10µl KOH 1N and 50µl potassium tetraborate 200mM are added to each tube and heated at 80°C for 30 minutes in a water bath. The reaction is cooled on ice for 5 minutes. 150µl of Ehrlich's reagent is added to the acetylated products and analyzed as described in methods.

Figure 9 is a graph illustrating the effect of time and temperature on the hydrolysis of UDP-N-acetylglucosamine to N-acetylglucosamine. To determine the temperature and time necessary for hydrolysis of UDP-N-acetylglucosamine to N-acetylglucosamine, 20nmoles of UDP-N-acetylglucosamine are incubated with HCl 0.1N at 20, 37, and 80°C for 5, 30, 60 and 120 minutes. 10µl of UDP-N-acetylglucosamine + 10µl HCl 1N + 80µl water are incubated in a microfuge tube for the times and temperatures indicated. 50µl potassium tetraborate 200mM is added for 30 minutes at 80°C followed by cooling on ice for 5 minutes. 130µl Ehrlich's reagent is added to each tube for 20 minutes at 37°C and the samples transferred to a 96 well microtiter plate to determine the OD at 585nm. A sample with 20nmoles of UDP-N-acetylglucosamine and no HCl 0.1N did not produce any N-acetylglucosamine indicating that HCl, or another suitable acidic solution, is required for hydrolysis of UDP-N-acetylglucosamine. Since no product was formed at 20°C or 37°C, these results indicate that a temperature greater than 37°C is required.

Figure 10 is a bar graph illustrating a comparison of the levels of UDP-N-acetylglucosamine obtained with the HPLC/MS method and the hydrolysis method described herein on extracts of COS cells transfected with GFAT-*beta*. The COS-GFAT-*beta* cells are plated on 35mm plates and when they reach 80% confluency the DMEM media is changed to DMEM plus 10% fetal bovine serum with no glucose for 16 hours. Glucose to a final concentration of 25mM is then added for 5 hours. The media is aspirated, the cells are washed in PBS 1X, scraped in 0.5ml of PBS, and transferred to a microfuge tube. The cells are centrifuged 2 minutes at 15000Xg at 4°C, and the pellet extracted in 200µl water + 200µl chloroform. The extract is sonicated for 15 seconds on ice using a microtip, and then centrifuged at 15000Xg for 5 minutes at 4°C and the supernatant saved. 10µl of the extract is taken for analysis by the HPLC/MS method. 150µl of the extract is placed in a microfuge tube + 50 µl acetonitrile + 20µl HCl 1N. This mixture is heated to 80°C for 10 minutes. 20µl KOH 1N is added + 50µl potassium tetraborate 200mM to each tube and heated at 80°C for 30 minutes, followed by cooling on ice for 5 minutes. 150µl of Ehrlich's reagent is added to each tube for 20 minutes at 37°C and 300µl is transferred to a 96 well microtiter plate for OD determination at 585nm. The nmoles of N-acetylglucosamine are calculated from a standard curve of N-acetylglucosamine.

While a number of embodiments of this invention have been represented, it is apparent that the basic construction can be altered to provide other embodiments that utilize the invention without departing from the spirit and scope of the invention. All such modifications and variations are intended to be included within the scope of the invention as defined in the appended claims rather than the specific embodiments that have been presented by way of example.

## Claims

1. A method for measuring the activity of glutamine:fructose 6-phosphate amidotransferase, which comprises the steps of:
(a) forming a mixture of glutamine:fructose 6-phosphate amidotransferase, fructose 6-phosphate, and glutamine;
(b) incubating the mixture in (a) under physiological conditions for a time sufficient to allow the formation of glucosamine 6-phosphate;
(c) acetylating the glucosamine 6-phosphate in (b) to form N-acetylglucosamine 6-phosphate;
(d) reacting the N-acetylglucosamine 6-phosphate in (c) with Ehrlich's reagent; and
(e) measuring the amount of N-acetylglucosamine 6-phosphate present in (d) by determining the optical density at 500-610nm of the mixture in (d) and comparing the amount of N-acetylglucosamine 6-phosphate in (d) with a control sample of N-acetylglucosamine 6-phosphate and a non-incubated control sample of glutamine:fructose 6-phosphate amidotransferase, or an incubated control sample of glutamine:fructose 6-phosphate amidotransferase without glutamine or without fructose 6-phosphate.

2. The method according to claim 1, wherein the glutamine:fructose 6-phosphate amidotransferase is glutamine:fructose 6-phosphate amidotransferase-human *alpha* form having a Km of 1.49mM for glutamine and a Km of 1.85mM for fructose 6-phosphate.

3. The method according to claim 1, wherein the glutamine:fructose 6-phosphate amidotransferase is glutamine:fructose 6-phosphate amidotransferase-human *beta* form having a Km of 1.99mM for glutamine and a Km of 6.8mM for fructose 6-phosphate.

4. The method according to any one of claims 1 to 3, wherein the mixture in (a) comprises glutamine:fructose 6-phosphate amidotransferase (0.25-25µl), glutamine (2-40mM), fructose 6-phosphate (2-40mM), phosphate buffered saline pH 7.4, ethylenediaminetetraacetic acid (5mM), and dithiothreitol (1mM).

5. The method according to any one of claims 1 to 4, wherein the incubation step in (b) is carried out at 37°C for a time period of 5 minutes to 5 hours.

6. The method according to any one of claims 1 to 5, wherein the acetylation step in (c) is carried out with acetic anhydride 0.00625 % to 0625 % in acetone followed by addition of potassium tetraborate.

7. The method according to any one of claims 1 to 5, wherein the acetylation step in (c) is carried out with acetic anhydride 0.09375 % in acetone followed by addition of potassium tetraborate.

8. The method according to any one of claims 1 to 7, wherein the Ehrlich's reagent in (d) comprises 2g p-dimethylaminobenzaldehyde, 0.3ml water, 2.2ml concentrated hydrochloric acid, and 17.4ml acetic acid, which is diluted 1:2 in acetic acid.

9. The method according to any one of claims 1 to 8, wherein the mixture in (a) is formed in a microtiter plate.

10. The method according to claim 9, further comprising the step of adding a control sample of N-acetylglucosamine 6-phosphate and a non-incubated control sample of glutamine:fructose 6-phosphate amidotransferase, or an incubated control sample of glutamine:fructose 6-phosphate amidotransferase without glutamine or without fructose 6-phosphate to the microtiter plate, after the incubation step in (b).

11. A method for measuring the inhibitory activity of a test compound on glutamine:fructose 6-phosphate amidotransferase, which comprises the steps of:
(a) forming a mixture of a test compound, glutamine:fructose 6-phosphate amidotransferase, fructose 6-phosphate, and glutamine;
(b) incubating the mixture in (a) under physiological conditions for a time sufficient to allow the test compound to inhibit glutamine:fructose 6-phosphate amidotransferase from forming glucosamine 6-phosphate;
(c) acetylating the glucosamine 6-phosphate in (b) to form N-acetylglucosamine 6-phosphate;
(d) reacting the N-acetylglucosamine 6-phosphate in (c) with Ehrlich's reagent; and
(e) measuring the amount of N-acetylglucosamine 6-phosphate in (d) by determining the optical density at 500-610nm of the mixture in (d) and comparing the amount of N-acetylglucosamine 6-phosphate in (d) with a control sample of glutamine:fructose 6-phosphate amidotransferase not containing the test compound and a control sample of N-acetylglucosamine 6-phosphate, thereby determining the inhibitory activity of the test compound.

12. The method according to claim 11, wherein the glutamine:fructose 6-phosphate amidotransferase is glutamine:fructose 6-phosphate amidotransferase-human *alpha* form having a Km of 1.49mM for glutamine and a Km of 1.85mM for fructose 6-phosphate.

13. The method according to claim 11, wherein the glutamine:fructose 6-phosphate amidotransferase is glutamine:fructose 6-phosphate amidotransferase-human *beta* form having a Km of 1.99mM for glutamine and a Km of 6.8mM for fructose 6-phosphate.

14. The method according to any one of claims 11 to 13, wherein the mixture in (a) comprises test compound, glutamine:fructose 6-phosphate amidotransferase (0.25-25µl), glutamine (10mM), fructose 6-phosphate (10mM), phosphate buffered saline pH 7.4, ethylenediaminetetraacetic acid (5mM), and dithiothreitol (1mM).

15. The method according to any one of claims 11 to 14, wherein the incubation step in (b) is carried out at 37°C for 1 hour.

16. The method according to any one of claims 1 to 15, wherein the acetylation step in (c) is carried out with acetic anhydride 0.00625 % to 0625 % in acetone followed by addition of potassium tetraborate.

17. The method according to any one of claims 11 to 15, wherein the acetylation step in (c) is carried out with acetic anhydride 0.09375% in acetone followed by addition of potassium tetraborate.

18. The method according to any one of claims 11 to 17, wherein the Ehrlich's reagent in (d) comprises 2g p-dimethylaminobenzaldehyde, 0.3ml water, 2.2ml concentrated hydrochloric acid, and 17.4ml acetic acid, which is diluted 1:2 in acetic acid.

19. The method according to any one of claims 11 to 18, wherein the mixture in (a) is formed in a microtiter plate.

20. The method according to claim 19, further comprising the step of adding a control sample of N-acetylglucosamine 6-phosphate and a non-incubated control sample of glutamine:fructose 6-phosphate amidotransferase, or an incubated control sample of glutamine:fructose 6-phosphate amidotransferase without glutamine or without fructose 6-phosphate to the microtiter plate, after the incubation step in (b).
